# EUROPEAN PATENT APPLICATION

(11) **EP 2 198 816 A1**
(43) Date of publication of application: **23.06.2010**
(21) Application number: 08829852.6
(22) Date of filing: 05.09.2008
(51) Int. Cl.: A61F 9/007

(54) **DRAINAGE DEVICE FOR OPHTHALMIC SURGERY**

(30) Priority: 07.09.2007 JP 2007232997
(71) Applicant: Yugen Kaisha Conan, Nerima-ku Tokyo 177-053 (JP); KABUSHIKI KAISHA TOP, Adachi-ku, Tokyo 120-0035 (JP)
(72) Inventor: SOYA, Koichi, Tokyo 177-0053 (JP); SOYA, Masahiro, Kawasaki-shi Kanagawa 211-0053 (JP)
(74) Representative: Henkel, Feiler & Hänzel
(86) International application number: PCT/JP2008/002459
(87) International publication number: WO 2009/031319

(57) **Abstract**

Provided is a drainage device for ophthalmic surgery wherein a good drainage ability can be continuously ensured by a simple structure without using an aspirator or the like. A drainage device (1) is composed of a main body (2), drooping members (3), a leg member (4) provided on a back surface of the main body (2), and a projecting member (5) projected into the palpebral fissure. The main body (2) and the drooping members (3) are made of paper, which has a wettable surface and prevents water from penetrating into the inside thereof, and molded integrally into a roughly trapezoidal shape as a whole. Lateral edges (23, 24) of the main body (2) are formed so as to follow the eyelid margin (E3) in the vicinity of the lateral angle of eye (E4). A gap (G) is formed between the main body (2) and a sterile seal (S). The drooping members (3) extend inward from the eyelid margin (E3). When liquid (L2) is pooled within the palpebral fissure (E5) and comes into contact with the bottom end of the drooping members (3), the liquid (L2) is drawn into the gap among the drooping members (3), the eyelid margin (E3) and the back surface of the main body (2) due to the wettability of the drooping members (3) (or the stickiness of the liquid (L 2)) and flows through the gap (G) between the main body (2) and the sterile seal (S) toward the downstream side. Thus, the liquid (L2) can be discharged from the palpebral fissure (E5) without using an aspirator or the like.

## Description

### PRIORITY CLAIM

The present application is based on and claims the priority benefit of Japanese Patent Application 2007-232997 filed on September 7, 2007, the contents of which are incorporated herein by reference in its entirety.

### BACKGROUND OF THE INVENTION

### Field of the Invention

The present invention relates to a drainage device used in an ophthalmic surgery for draining out liquid pooled in the palpebral fissure (recession on the eyelid) of a patient lain with face up.

### Description of the Related Art

An ophthalmic surgery under a microscope is generally operated on a patient when the patient is laid with face up and the patient's face is covered by sterile unwoven fabrics with only an eyeball to be operated and the corresponding eyelid exposed. In most cases, the entire part of the patient's face except the eyeball is further covered with a sterile seal to envelope the eyelid margin and lashes. Eye-blinking is usually prevented by an eyelid speculum. In the ophthalmic surgery such as a cataract operation, in order to protect the eyeball tissues and assure a clean field of operation, the operation is performed with physiological saline solution to be dropped on the eyeball continuously. Therefore, the physiological saline solution and the like will be pooled around the margin of the cornea (the conjunctiva) in the palpebral fissure.

Moreover, in an intraocular surgery, in order to maintain the intraocular pressure, an intraocular viscoelastic liquid with high viscosity or an intraocular infusion is injected or replenished continuously. In such intraocular surgery, when inserting a surgical instrument into the eyes or taking the surgical instrument out of the eyes and due to the strain (wound dehiscence) caused by an operation in the intraocular surgery, the intraocular liquid will flow out in a large amount and be pooled in the palpebral fissure.

After the liquid such as the intraocular liquid and the intraocular infusion pooled in the palpebral fissure wets the cornea and the conjunctiva of high hydrophilicity, it will drop from the lowest portion, namely the lateral angle of eye (the lateral canthus), to the outside because of the attraction of gravity after overflowing the lateral angle of eye.

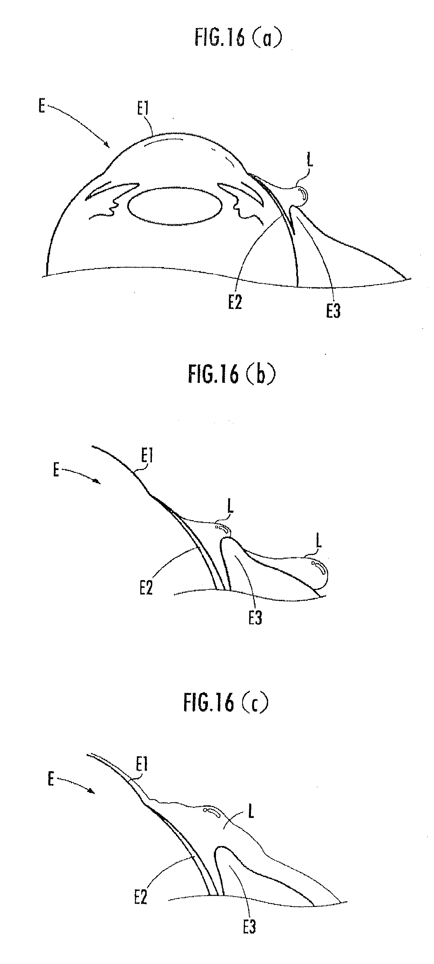

However, as illustrated in Fig. 16(a), it is quite often that the eyelid margin E3 bulges over the lateral angle of eye of the eyeball E of a patient, or it is quite often that the ambient of the lateral angle of eye is covered with the sterile seal (not shown) as mentioned above. The eyelid margin E3 and the sterile seal serves as a dam to pool the liquid due to a lower hydrophilicity in comparison with the conjunctiva E2, and the liquid level of the liquid L rises due to the surface tension, if the liquid level reaches the field of operation, it will affect the clearness of the field of operation, making it difficult to perform the surgical operation. Especially, when an eyelid speculum is set on an eye with a narrow opening which is called as a small eye, the narrower the eye opening is, the eyelid margin E3 and the skin of the lateral angle of eye will bulge higher like a wall. Thereby, the liquid level will rise to reach the cornea E1 which is the field of operation, disturb the clearness of the field of operation due to the liquid bumping and thus become a big trouble to the surgical operation.

After the liquid level of the pooled liquid in the palpebral fissure rises to the highest height according to the surface tension, the liquid pooled more than that can not be held will drop out. However, even after the liquid has dropped out, the liquid level will remain bumped due to the surface tension of the liquid pooled in the palpebral fissure. As illustrated in Fig. 16(b), despite the fact that the bumped liquid level is remained lower than the highest height, it might disturb the clearness of the field of operation, making it difficult to perform the surgical operation.

On the other hand, when the liquid is continuously supplied into the palpebral fissure, the supplied liquid will flow out continuously from the lateral angle of eye; however, as illustrated in Fig. 16(c), the liquid flowing out from the lateral angle of eye, although the liquid level is lower than the highest height, will disturb the clearness of the field of operation. When the liquid is flowing out continuously, particularly the liquid pooled in the eyes undulating against each other will give birth to diffuse reflection, which will become a big problem deteriorating the surgical operation. Even the liquid stops flowing continuously, similar to the case illustrated in Fig. 16(b), the clearness of the field of operation may be deteriorated by the liquid pooled in the palpebral fissure.

In order to solve the problem of the clearness of the field of operation being deteriorated by the liquid pooled in the palpebral fissure, the following approaches have been adopted to drain the pooled liquid to the outside of eyes (the palpebral fissure). Firstly, there has been considered to dispose a hygroscopic material, such as a piece of gauze or a spongy element on the eyelid margin of the lateral angle of eye, suspending from the lateral angle of eye downward in the direction of ear to reduce the liquid level from rising around the eyelid margin of the lateral of eye or the sterile seal due to the low hydrophilicity thereof. For example, there has been disclosed a liquid absorbing device for a surgical operation in Japanese Patent Laid-open No. H8-317939 (hereinafter, referred to as Patent Document 1). The liquid absorbing device is provided with a contact absorbing member for absorbing liquid in the palpebral fissure such as a fiber (a piece of gauze or the like) or a sponge, an absorbing main body for pooling the liquid, and a liquid guiding member for joining the contact absorbing member and the absorbing main body.

However, according to the above-mentioned approach disclosed in Patent Document 1 to drain the pooled liquid to the outside of the eye (the palpebral fissure) through soaking the liquid with a piece of gauze or the like, if there is a large amount of liquid leaking out, the liquid will flow over the surface of the gauze or the like, therefore, it is impossible for it to inhibit sufficiently the liquid level from bumping low. Moreover, since the spongy element or the like is slow in soaking liquid, therefore, it is impossible for it to drain liquid (water) instantly.

In order to maintain the eyeball in shape, a transparent liquid of high viscosity is flown into the eyes in a cataract operation or the like. However, the liquid of high viscosity overflows to the outside of the eyes during the cataract operation, the gauze, the spongy element or the like will be clogged by the liquid of high viscosity and the drainage effect will be deteriorated dramatically. In this case, even though it has been tried to remove the liquid of high viscosity to recover the drainage effect, since the liquid of high viscosity has been absorbed inside the gauze or the spongy element, it cannot be removed according to simple cleaning, therefore, it is difficult to recover the drainage effect. Moreover, it is often that blood will be blended into the liquid in the palpebral fissure in a surgical operation, and the blood will coagulate in the spongy element. The coagulated blood will deteriorate the absorbing ability of the spongy element and also it would be difficult to wash out the coagulated blood therefrom.

Further, according to the absorbing device disclosed in Patent Document 1, when disposing the absorbing device after the eyelid speculum has been set on the patient, it is necessary to pass the absorbing device under through the eyelid speculum, which makes the disposal work troublesome. In addition, if the absorbing device happens to have a contact with the eyelid speculum in the disposal work, it may apply unpleasant feeling to the patient.

With respect to the treatment of a small eye, there has been used such a device (not shown) that a spindle is hanged to the eyelid margin of the lateral angle of eye to pull down the eyelid margin and the wall of skin. However, since the device cannot make the eyelid margin of the lateral angle of eye and the wall of skin disappear completely, although it could reduce the amount of liquid pooled in the palpebral fissure, the effect thereof is not sufficient since the liquid may still be remained in the palpebral fissure. Additionally, since there is a limit in extending the skin, therefore, the mentioned device has an adverse effect involved in extending the skin by giving birth to a painful feeling to the patient.

There has been also disclosed an eyelid speculum (not shown). The eyelid speculum is configured to be hollow inside and a plurality of small holes are opened at a portion disposed in the palpebral fissure, the liquid pooled in the palpebral fissured is sucked up through the plurality of small holes with an aspirator connected to a bottom end portion of the eyelid speculum. However, according to the eyelid speculum of this kind, it is necessary to dispose an electric aspirator; therefore, it is not simple in structure and consequently not convenient in use. Further, it is troublesome to use the drainage device since it is necessary to clean or sterilize the drainage device. Furthermore, when the eyelid speculum is in use, the plurality of small holes contact the conjunctiva, it will be absorbed by the conjunctiva, and consequently, it will be clogged by the conjuctiva, which makes it to have insufficient drainage ability. Moreover, this kind of device has such a shortcoming as to cause damage on tissues.

Also, there has been disclosed in Japanese Patent Laid-open No. 2000-60895 (hereinafter, referred to as Patent Document 2) a drainage device provided with a flat tubular member which is curved according to the shape of an eyeball and a curved pipe extended from the inner side of the tubular member to the outside. According to Patent Document 2, the cylindrical member of the drainage device is inserted between the eyelid of the lateral angle of eye and the conjunctiva, an exit of the pipe is connected with an electric aspirator to suck the liquid pooled in the tubular member through the pipe.

However, according to the drainage device of this type, it is necessary to provide an electric aspirator; therefore, it is not simple in structure and consequently not convenient in use. Further, it is troublesome to use the drainage device since it is necessary to clean or sterilize the drainage device. Since the tubular member is inserted between the eyelid of the lateral angle of eye and the conjunctiva, therefore, it is possible to damage the conjunctiva or the like; meanwhile, the tubular member applies a pressure to the eyeball, varying the inner pressure of the eyeball (the intraocular pressure), and thus, it is possible to affect the operation itself.

### SUMMARY OF THE INVENTION

The present invention has been accomplished in view of the aforementioned problems, and it is therefore an object of the present invention to provide a drainage device for an ophthalmic surgery capable of maintaining good drainage ability continuously in a simple structure without using an aspirator or the like.

To attain an object described above, there is provided in the present invention a drainage device used in an ophthalmic surgery to drain a liquid to the outside from the palpebral fissure of a patient lain with face up. The drainage device of the present invention comprises: a planar main body configured to be disposed with a predefined gap on a supporting surface on the eyelid in the vicinity of the lateral angle of eye; the main body is made of a water-proof material and has a wettable back surface; the main body is provided with a projecting member projected from an edge of the palpebral fissure into an inner side thereof; and when the main body is disposed on the supporting surface, the liquid pooled in the palpebral fissure is adhered by a back surface of the projecting member and is drained into the gap between the main body and the supporting surface, and the drained liquid flows through the gap to be discharged to the outside of the main body.

According to the drainage device for ophthalmic surgery of the present invention, when the liquid pooled in the palpebral fissure in an ophthalmic surgery contacts the back surface of the projecting member provided in the main body, the liquid is adhered by the back surface of the projecting member, the liquid is drained into the gap between the back surface of the main body and the supporting surface due to the wetting (or adhering force). Thereafter, the liquid advances in the gap between the main body and the supporting surface toward the downstream side mainly according to the adhering force of the liquid and the elevation difference in the longitudinal direction of the main body to the end portion of the main body. Herein, it is considered that a capillary phenomenon is additionally contributing to the advance of the liquid. After the liquid pooled at the end portion of the main body is turned into drops, it drops downward according to the pushing from the after liquid discharged from the projecting member and the attraction of gravity.

According to the drainage device for ophthalmic surgery of the present invention, since the main body is planar, the liquid pooled in the palpebral fissure can be discharged to the outside in a simple structure without the need of either a tubular member or a pipe, or an aspirator used in the conventional drainage device.

Since the liquid flows between the back surface of the main body and the supporting surface, the liquid level of the liquid flowing out from the eyelid margin can be reduced. Compared with the situation that the liquid is discharged through soaking the liquid with a piece of gauze or a sponge as in the conventional arts, the resistance to the flow of the liquid is extremely small when the liquid is made to flow between the back surface of the main body and the supporting surface. Therefore, the drainage device of the present invention is fast in draining liquid, it can discharge the liquid to the outside fast and certainly even when the liquid is continuously administered into the palpebral fissure. Moreover, the main body is made of a water-proof material, which makes it different from the gauze or the sponge, even the main body is adhered with the liquid of high viscosity or coagulated with blood at the surface thereof, since the blood or the like is difficult to be soaked into the inner side of the main body, it is easy to be washed off. Thereby, even in the surgical operation, it is possible for an operator or an assistant to clean the drainage device appropriately to maintain it working in high drainage ability.

The drainage device of the present invention can be used by disposing the planar member on the eyelid only, thus, it will not apply a pressure to the eyeball. The wettability of the back surface of the main body may be realized through using a material having wettability in the main body or through processing the main body without wettability to have wettability.

It is preferable that the drainage device for ophthalmic surgery of the present invention has a drooping member drooped toward the conjunctiva in the vicinity of the lateral angle of eye provided in the projecting member. By providing the drooping member, the liquid in a lower portion including the bottom of the palpebral fissure can be contacted by the drooping member. Therefore, the liquid even in the vicinity of the bottom of the palpebral fissure can be drained out by the drooping member. Since the drooping member is drooping toward the conjunctiva in the vicinity of the lateral angle of eye, the drooping member can be engaged to the edge portion of the palpebral fissure. Accordingly, when the main body is placed on the supporting surface, the position thereof can be easily determined by the drooping member. Since the main body can be prevented from deviating from the supporting surface to the downstream side (downward to the direction of the patient's ear) by the drooping member, it would be difficult for the main body to be drifted to the downstream side by the liquid flowing out from the palpebral fissure.

It is preferable that the drainage device for ophthalmic surgery of the present invention has a part of or the entire part of the main body inclined to the supporting surface when viewed from the flowing direction of the liquid. Accordingly, by inclining the main body, the inclined portion can have a narrow space and a wide space formed in the gap between the back surface of the main body and the supporting surface. For example, when the liquid drained from the palpebral fissure is of a smaller amount, the liquid flows through the narrow space in the gap. Therefore, when the liquid drained from the palpebral fissure is of a smaller amount, since there is no break in the flow of the liquid, the liquid can be drained out from the palpebral fissure continuously. On the other hand, when the liquid drained from the palpebral fissure is a greater amount, the liquid expands from the narrow space to the wide space and flows out. Therefore, when the liquid drained from the palpebral fissure is of a greater amount, the liquid can flow out smoothly. It is acceptable for the inclined surface to have a planar shape, to be curved, or to be inclined stepwise.

When the main body is inclined in the drainage device for ophthalmic surgery of the present invention, it is acceptable for the main body to protrude in a mountain-like shape when viewed from the flowing direction of the liquid or to have a capitalized letter "V" shape when view from the flowing direction of the liquid.

It is preferable that the drainage device for ophthalmic surgery of the present invention to provide a plurality of slits orthogonal to the flowing direction of the liquid in the main body. When the slits are provided in the main body, the main body is split by the slits into a strip. Thus, even though the supporting surface is uneven, the main body can bend to shape with the uneven supporting surface. Therefore, even though the supporting surface is uneven, the gap between the supporting surface and the min body can be prevented from becoming too wide, thereby, the liquid can be discharged from the palpebral fissure with certainty.

It is preferable that the drainage device for ophthalmic surgery of the present invention to have a leg member with a predefined height provided on the back surface of the main body. By providing the leg member, the gap between the main body and the supporting surface can be maintained. Moreover, the main body can be inclined via the leg member.

It is preferable that the drainage device for ophthalmic surgery of the present invention to have an edge of the projecting member curved in accordance with the eyelid margin provided in the main body. According thereto, when the liquid level of the liquid in the palpebral fissure increases, the location which can be used to drain out the liquid is expanded to the edge, the increment of the liquid level of the liquid flowing out from the palpebral fissure can be inhibited by the entire edge.

It is preferable that the drainage device for ophthalmic surgery of the present invention to have a hydrophilic membrane provided on the back surface of the main body toward the flowing direction of the liquid with better wettability than the other surface thereof. According thereto, since the liquid contacting the back surface of the main body uses the hydrophilic membrane as a flowing base to flow between the main body and the supporting surface downstream, thereby, the liquid can flow toward the downstream of the main body smoothly. When the liquid from the palpebral fissure becomes temporarily less, the liquid flowing between the back surface of the main body and the supporting surface may become intermittent due to the reason that the liquid flowing from the main body to the outside drops with some vigor or is temporarily vaporized. Under this situation, even if the liquid from the palpebral fissure is increased again, when the liquid contacting the back surface of the main body passes between the main body and the supporting surface, the interface generated due to the intermittent liquid and the surface tension of the liquid is needed to be broken. However, according to the present invention, when the hydrophilic membrane is provided in the main body, it will be difficult for the liquid between the main body and the supporting surface to become intermittent due to the reduction of the liquid from the palpebral fissure; even though the liquid becomes intermittent, since the resistant to break the interface is decreased by the hydrophilic membrane, the liquid can be discharged instantly.

Thereby, it is preferable that the hydrophilic membrane is disposed at the narrow space of the gap in the inclined portion. When the liquid drained from the palpebral fissure becomes less, the liquid will flow through the narrow space of the gap. By disposing the hydrophilic membrane at the narrow space of the gap, even though the liquid drained from the palpebral fissure is less, the liquid can be made to flow out smoothly. In addition, the gap has a better holding ability for holding the liquid when the liquid is motionless.

It is preferable that the drainage device for ophthalmic surgery of the present invention to have a hygroscopic member provided on the back surface of the main body toward the flowing direction of the liquid. According thereto, the similar effect can be obtained to the case where the hydrophilic membrane is provided. It is difficult for the liquid absorbed by the hydroscopic member to be vaporized, therefore, compared with the hydrophilic membrane, the liquid can be held for a longer time between the main body and the supporting surface. Therefore, it is difficult for the liquid to become intermittent, when the liquid in the palpebral fissure is decreased and thereafter increased again, it is possible to increase the liquid discharging amount instantly in follow of the increment of the liquid.

It is preferable that the drainage device for ophthalmic surgery of the present invention to have a fixing member protruded to the vicinity of the eyelid margin to be fixed on the eyelid in the main body. It is acceptable to provide an engaging member protruded to the palpebral fissure to be engaged to the eyelid margin in the main body. By providing the fixing member or the engaging member, the drainage device of the present invention can be fixed to the vicinity of the patient's eyeball with certainty, as a result thereof, the liquid can be discharged to the outside with certainty during the ophthalmic surgery. Moreover, in order to fix the drainage device of the present invention to the vicinity of the patient' s eyeball with certainty, it is also acceptable that the drainage device for ophthalmic surgery of the present invention to have an adhesive member provided on the backside of the main body for adhering to the supporting surface.

It is acceptable that the main body of the drainage device for ophthalmic surgery of the present invention is composed of a holding member to be fixed on the supporting surface and an inclination member inclined obliquely upward from the holding member. Specifically, the holding member may be fixed on the supporting surface and the liquid may be discharged to the outside of the main body by flowing through the gap between the holding member and the inclination member. According thereto, even if the supporting surface is uneven, the boundary between the holding member and the inclination member is assured to be continuous, as a result thereof, the liquid in the palpebral fissure can be discharged to the outside with certainty.

It is preferable that the drainage device for ophthalmic surgery of the present invention to have a portion of the drooping member opposite to the eyelid margin inclined toward the eyelid margin when the main body is disposed on the supporting surface. According thereto, the main body can be easily engaged to the eyelid margin by the drooping member, therefore, the drainage device of the present invention can be fixed with certainty at the vicinity of the patient's eyeball. Since the inclination of the drooping member can generate with certainty a gap between the drooping member and the eyelid margin, it is easy to give birth to a capillary phenomenon due to the adhesive force of the liquid in the drooping member.

It is preferable that the drainage device for ophthalmic surgery of the present invention to have a plurality of drooping members disposed in parallel with an interval therebetween appropriate for giving birth to a capillary phenomenon between the plurality of drooping members. According thereto, the adhesive area of the liquid between the plurality of drooping members is increased, which makes it easy to give birth to a capillary phenomenon, and as a result thereof, the liquid pooled in the palpebral fissure can be discharged to the outside instantly.

It is preferable that the drainage device for ophthalmic surgery of the present invention to have the drooping member formed with an upstream side and a downstream side symmetrical at the central position of the main body in a longitudinal direction identical to the flowing direction of the liquid. According thereto, for example, the drooping member at one side may be used when the drainage device of the present invention is applied to the right eye of the patient; on the other hand, the drooping member at the other side may be used when the drainage device of the present invention is applied to the left eye of the patient. Moreover, the disposed position of the drainage device may be different according to which is the dominant hand of an operator. Even in this situation, it is possible to dispose the drainage device of the present invention with only one shape at the most convenient position for the operator to perform the surgery.

It is preferable that the drainage device for ophthalmic surgery of the present invention to have the main body formed to have a size to be disposed inside a supporting member of a capitalized letter "C" shape in an eyelid speculum. According thereto, when the drainage device of the present invention is disposed on the supporting surface, it is not necessary to pass under the eyelid speculum as the conventional arts do. Therefore, it is not necessary to pass the drainage device under the eyelid speculum even after the eyelid speculum has been disposed on the patient, which makes it easy to hold the drainage device of the present invention on the supporting surface.

### BRIEF DESCRIPTION OF THE DRAWINGS

Fig. 1(a) and Fig. 1(b) are explanatory diagrams illustrating a drainage device for ophthalmic surgery according to a first embodiment of the present invention;
Fig. 2 is a diagram illustrating an applied state of the drainage device of the first embodiment;
Fig. 3 is a diagram illustrating an applied state of the drainage device of the first embodiment;
Fig. 4 is a diagram illustrating an applied state of the drainage device of the first embodiment;
Fig. 5(a) to Fig. 5(c) are explanatory diagrams illustrating a drainage device for ophthalmic surgery according to a second embodiment of the present invention and an applied state thereof, respectively;
Fig. 6(a) and Fig. 6(b) are explanatory diagrams illustrating a drainage device for ophthalmic surgery according to a third embodiment of the present invention and an applied state thereof, respectively;
Fig. 7(a) and Fig. 7(b) are explanatory diagrams illustrating a drainage device for ophthalmic surgery according to a fourth embodiment of the present invention and an applied state thereof, respectively;
Fig. 8(a) and Fig. 8(b) are explanatory diagrams illustrating a drainage device for ophthalmic surgery according to a fifth embodiment of the present invention and an applied state thereof, respectively;
Fig. 9(a) and Fig. 9(b) are explanatory diagrams illustrating a drainage device for ophthalmic surgery according to a sixth embodiment of the present invention and an applied state thereof, respectively;
Fig. 10 is an explanatory diagram illustrating a drainage device for ophthalmic surgery according to a seventh embodiment of the present invention;
Fig. 11 is an explanatory diagram illustrating a drainage device for ophthalmic surgery according to an eighth embodiment of the present invention;
Fig. 12(a) and Fig. 12(b) are explanatory diagrams illustrating a drainage device for ophthalmic surgery according to a ninth embodiment of the present invention;
Fig. 13(a) and Fig. 13(b) are explanatory diagrams illustrating a drainage device for ophthalmic surgery according to a tenth embodiment of the present invention;
Fig. 14(a) and Fig. 14(b) are explanatory diagrams illustrating a drainage device for ophthalmic surgery according to an eleventh embodiment of the present invention;
Fig. 15(a) to Fig. 15(e) are explanatory diagrams illustrating various variation examples of a drooping member, respectively; and
Fig. 16(a) to Fig. 16(c) are explanatory diagrams illustrating states where a liquid is pooled in a palpebral fissure of a patient, respectively.

### DETAILED DESCRIPTION OF THE PREFERRED EMBODIMENTS

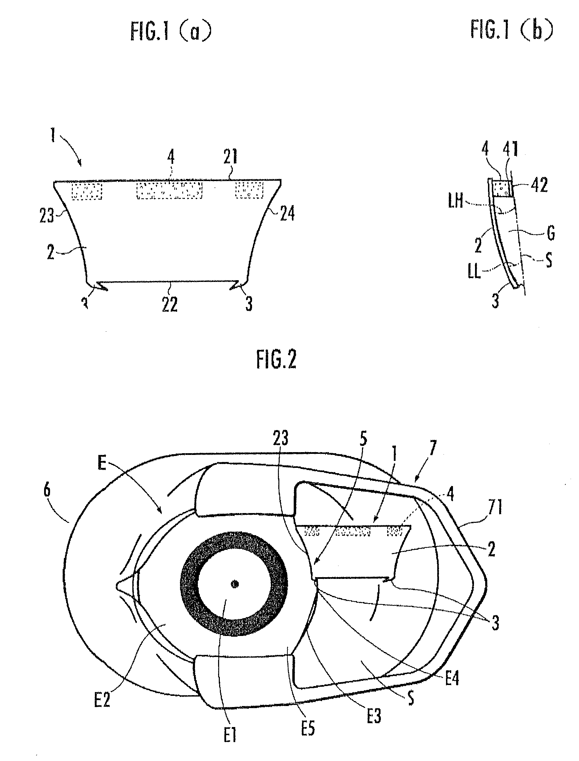

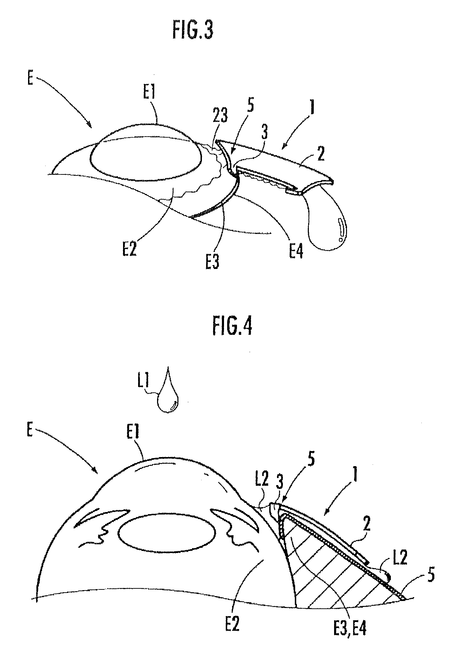

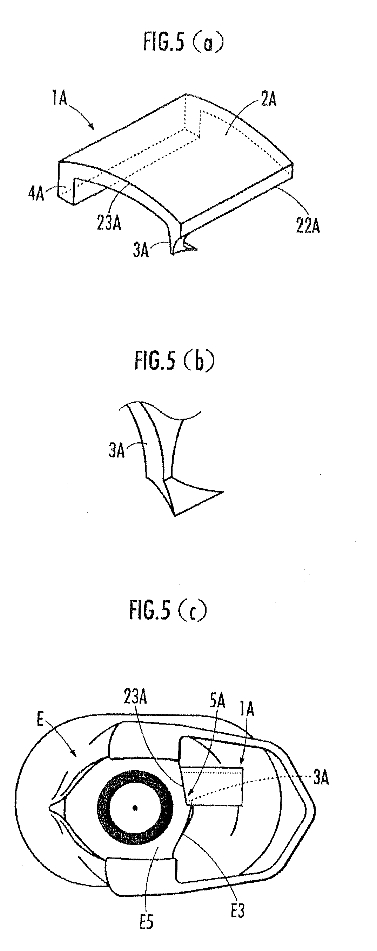

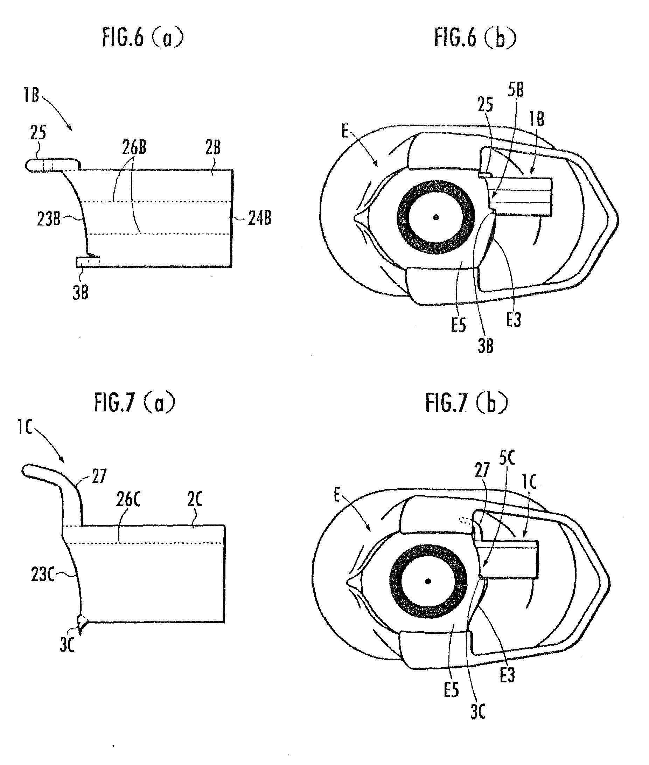

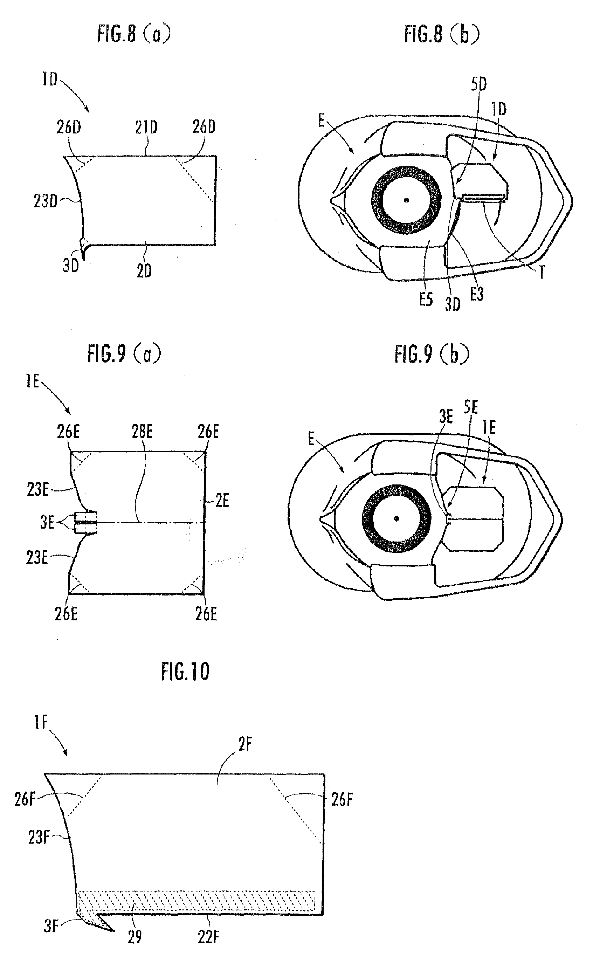

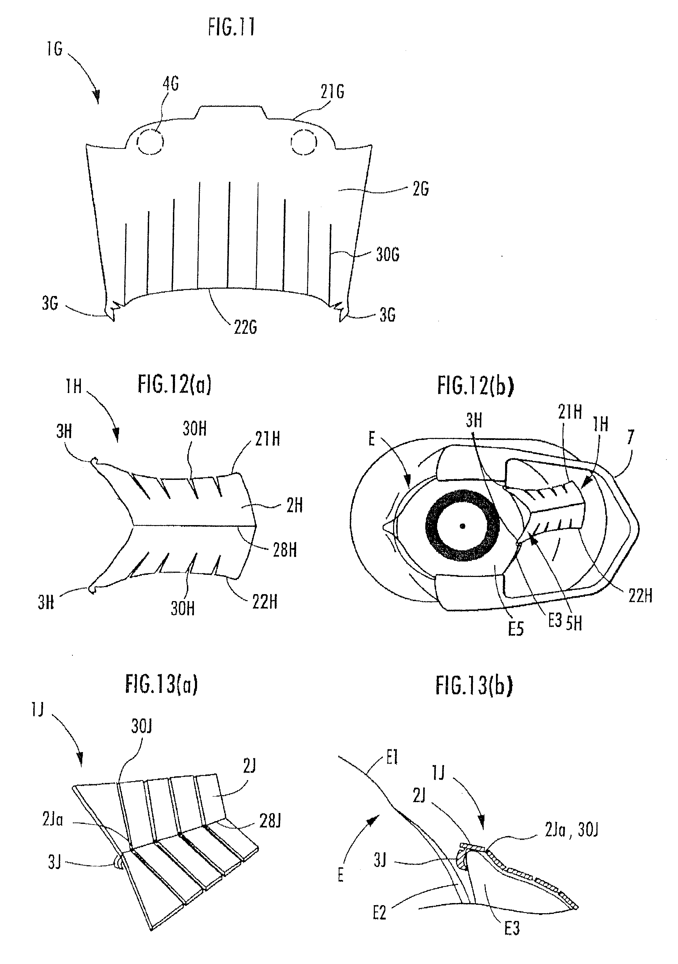

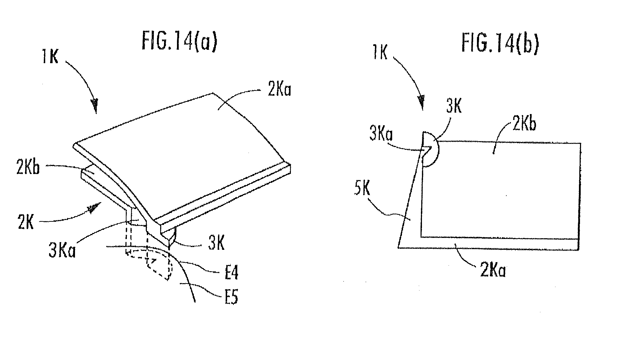

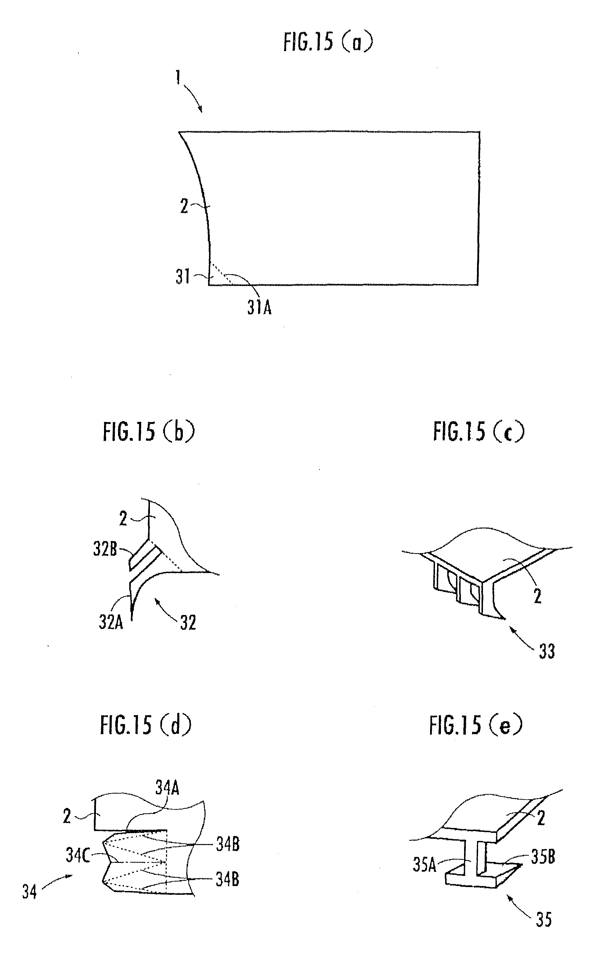

Hereinafter, an embodiment of to the present invention will be described in detail with reference to the drawings. Fig. 1 (a) and Fig. 1(b) are explanatory diagrams illustrating a drainage device for ophthalmic surgery according to a first embodiment of the present invention; Fig. 2 is a diagram illustrating an applied state of the drainage device of the first embodiment; Fig. 3 is a diagram illustrating an applied state of the drainage device of the first embodiment; Fig. 4 is a diagram illustrating an applied state of the drainage device of the first embodiment; Fig. 5(a) to Fig. 5(c) are explanatory diagrams illustrating a drainage device for ophthalmic surgery according to a second embodiment of the present invention and an applied state thereof, respectively; Fig. 6(a) and Fig. 6(b) are explanatory diagrams illustrating a drainage device for ophthalmic surgery according to a third embodiment of the present invention and an applied state thereof, respectively; Fig. 7 (a) and Fig. 7 (b) are explanatory diagrams illustrating a drainage device for ophthalmic surgery according to a fourth embodiment of the present invention and an applied state thereof, respectively; Fig. 8(a) and Fig. 8(b) are explanatory diagrams illustrating a drainage device for ophthalmic surgery according to a fifth embodiment of the present invention and an applied state thereof, respectively; Fig. 9(a) and Fig. 9(b) are explanatory diagrams illustrating a drainage device for ophthalmic surgery according to a sixth embodiment of the present invention and an applied state thereof, respectively; Fig. 10 is an explanatory diagram illustrating a drainage device for ophthalmic surgery according to a seventh embodiment of the present invention; Fig. 11 is an explanatory diagram illustrating a drainage device for ophthalmic surgery according to an eighth embodiment of the present invention; Fig. 12(a) and Fig. 12(b) are explanatory diagrams illustrating a drainage device for ophthalmic surgery according to a ninth embodiment of the present invention; Fig. 13(a) and Fig. 13(b) are explanatory diagrams illustrating a drainage device for ophthalmic surgery according to a tenth embodiment of the present invention; Fig. 14(a) and Fig. 14(b) are explanatory diagrams illustrating a drainage device for ophthalmic surgery according to an eleventh embodiment of the present invention; and Fig. 15(a) to Fig. 15(e) are explanatory diagrams illustrating various variation examples of a drooping member, respectively.

As illustrated in Fig. 1, a drainage device 1 according to a first embodiment of the present invention is comprised of a main body 2, a drooping member 3, and a leg member 4 disposed at a back surface of the main body 2. The main body 2 and the drooping member 3 are integrally made of paper into a roughly trapezoidal shape. The paper used is processed in such a way that it has a better wettability then human skin or a sterile seal and cannot be penetrated by liquid. As an example of the material used in the main body 2, "Tyvek" (registered trade mark) by Du Pont or the like may be given. The lateral direction in Fig. 1(a) is the flowing direction of liquid. In the present specification, the lateral direction is described as the longitudinal direction. Moreover, in the present specification, the vertical direction in Fig. 1(a) is described as the width direction.

As illustrated in Fig. 1(a), the upper side 21 of both sides of the main body 2 in the longitudinal direction is configured to be longer than the lower side 22 thereof. As illustrated in Fig. 1(a), the lateral edges 23 and 24 in the width direction are configured to have a curvature toward the inner side from the upper side to the lower side. The curvature of the lateral edge in the width direction is configured to match with that of the eyelid margin E3 in the vicinity of the lateral angle of eye E4 of a patient (refer to Fig. 2). Moreover, the main body 2 is provided with a projecting member 5 projected into the palpebral fissure E5.

As illustrated in Fig. 1(b), the main body 2 is fixed by the leg member 4 on a supporting surface with an inclination to the supporting surface, and is preliminarily formed with a curvature gently projected toward the outer surface thereof. When the drainage device 1 with the above-mentioned configuration is placed on the eyelid or a sterile seal S illustrated with a dashed straight line in Fig. 1(b), it forms a gap G with the supporting surface. The gap G has a roughly triangular shape when viewed from the flowing direction of liquid.

The drooping member 3 is disposed at the projecting member 5 of the main body 2, integral with the main body 2. In the present embodiment, the drooping members 3 extend from both sides of the lower side of the main body 2 obliquely inward. As illustrated in Fig. 1(a), the drooping member 3 has a roughly triangular shape in profile. When the drainage device 1 is in use, either one of the drooping members 3 is bent. In a surgical operation, according to either the left eyeball or the right eyeball to be operated or the standing position of the operator with respect to the eye, it is necessary to change the disposing position of the drainage device 1. Therefore, according to the present embodiment, the drooping members 3 are disposed at two locations, making the drainage device 1 of the present invention possible to be used in various situations.

The leg member 4 is made of flexible resin foam, and is fixed at the back surface in the vicinity of the upper side 21 of the main body 2 by an adhesive agent. The disposing surface of the leg member 4 is provided with an adhesive layer 41. The outer surface of the adhesive layer 41 is covered with a release paper 42.

The outer surface of the drainage device 1 of the present embodiment is colored similarly to the skin color of a patient for the purpose of avoiding uncomfortable feeling to be applied to an operator in a surgical operation. In order to prevent light rays reflected from the illumination from entering the eyelids in the surgical operation, the back surface of the drainage device 1 is colored to matte-black.

According to the drainage device 1 of the present embodiment, it is necessary to apply wettability to the back surface side of the main body 2 and the projecting member 5 thereof. When used, the main body 2 will be pulled by an adhesive force from a liquid L2 toward the supporting surface side. In order to ensure the gap G when the main body 2 is adhered to the supporting surface, it is necessary for the main body 2 to be processed so that the liquid cannot penetrate to the inner side and to have a rigidity of some extent. In order to prevent the drainage device 1 from moving against the intention of an operator when used, the stability of disposing the drainage device 1 on the supporting surface is required. The drainage device 1 of the present embodiment satisfies the above-mentioned requirement with the characteristics and thickness of the paper serving as the raw material of the main body 2, the shape of the curvature projected toward the outer surface of the main body 2, and the adhesive layer 41 of the leg member 4.

Hereinafter, the usage of the drainage device 1 according to the first embodiment will be described with reference to Fig. 2. The eye of a patient, except the eyeball E is covered by the sterile seal S with the eyelid margin and the eyelashes enclosed therein, the ambient of the eyeball E is covered by unwoven fabrics 6. As illustrated in Fig. 2, the palpebral fissure E5 is enlarged and fixed by an eyelid speculum 7 provided with a supporter 71 of a roughly "C" shape to prevent the eye from blinking. Thereby, the cornea E1 and the conjunctiva E2 of the eyeball E are exposed.

The drainage device 1 is placed and fixed on the outer surface of the sterile seal S in the vicinity of the lateral angel of eye E4 of the patient. In Fig. 2, the drainage device 1 is placed at a location toward the head side (upper side in Fig. 2) from the center of the eyeball of the patient. As illustrated in Fig. 2, the drooping member 3 at the left side is folded and inserted to the inner side (lower side) of the palpebral fissure E5. The release paper 42 on the outer surface of the leg member 4 is released to adhere the adhesive layer 41 to the outer surface of the sterile seal S, thereby, the leg member 4 is fixed. As illustrated in Fig. 3 and Fig. 4, the drooping member 3 is extended from the eyelid margin E3 into the inside, the edge of the drooping member 3 opposite to the eyelid margin E3 is engaged to the eyelid margin E3.

For example, when a cataract operation is performed on the patient, an ophthalmic solution L1 is intermittently dropped to the eye from the above of the eyeball E. Consequently, a liquid L2 including the ophthalmic solution L1 is pooled on the conjunctiva E2 in the palpebral fissure E5. When the liquid L2 has a contact with the drooping member 3, due to the wettability of the outer surface of the drooping member 3 (or the adhesive force of the liquid L2), the liquid level of the liquid L2 rises along the outer surface of the drooping member 3. As illustrated in Fig. 1(a), since the drooping member 3 is extended obliquely toward the inner side of the main body 2, an interval is formed between the drooping member 3 and the eyelid margin E3.

Thereby, the capillary phenomenon occurs easily. Owning to the adhesive force of the liquid L2 and the capillary phenomenon, the liquid level of the liquid L2 rises along the interval between the drooping member 3 and the eyelid margin E3, and the liquid L2 contacts the back surface of the main body 2. Since the back surface of the main body 2 has wettability, the liquid L2 penetrates into the gap G between the back surface of the main body 2 and the outer surface of the sterile seal S and flows to the downstream through the gap G between the main body 2 and the sterile seal S. The reason thereof has been considered to be related to the adhesive force of the liquid, the elevation difference in the longitudinal direction of the main body, the capillary phenomenon, and the siphon effect occurred due to the liquid level of the liquid L2 and the elevation difference in the longitudinal direction of the main body. Thereafter, as illustrated in Fig. 4, the liquid L2 reached to the end portion of the downstream of the main body 2 is turned into drops at the end portion of the downstream of the main body 2, it drops downward to (ear side of the patient) the outside of the main body 2 due to the attraction of gravity.

Thus, the liquid L2 including the ophthalmic solution L1 in the palpebral fissure E5 and the liquid flowing out from the eye has been drained to the outside through the drainage device 1, thereby, a flow of the liquid L2 from the palpebral fissure E5 to the outside of the drainage device 1 is formed. Therefore, even though the ophthalmic solution L1 is continuously administered into the palpebral fissure E5, the liquid L2 flows out continuously from the palpebral fissure E5 to the outside of the drainage device 1. Accordingly, the liquid level of the liquid L2 in the palpebral fissure E5 will be maintained below the height of the main body 2. Moreover, since the liquid L2 is being pulled on the back surface of the projecting member 5 due to the wettability of the back surface, the liquid level of the liquid L2 in the palpebral fissure E5 can be maintained low.

When the ophthalmic solution L1 administered to the palpebral fissure E5 or the liquid flowing out from the eye becomes more, as a result thereof, the liquid L2 in the palpebral fissure E5 will become more, the flow of the liquid L2 changes from the state of LL to the state of LH illustrated by two-dot chain lines, respectively, in Fig. 1(b), for example. Since the main body 2 is oblique to the supporting surface as illustrated in Fig. 1(b), the amount of liquid flowing on the back surface of the main body 2 increases. On the other hand, when the ophthalmic solution L1 administered to the palpebral fissure E5 becomes less and consequently the liquid L2 in the palpebral fissure E5 will become less, the flow of the liquid L2 in the figure becomes the state of LL as illustrated in Fig. 1(b), for example. Thereby, even though the amount of liquid L2 flowing through the back surface of the main body 2 becomes less, since the back surface of the main body 2 and the supporting surface are closer to each other at some locations, the liquid L2 can be maintained to flow through the main body 2 and the supporting surface without becoming intermittently.

Even when the liquid L2 to be drained temporarily vanishes and the flow stops, the liquid L2 can be easily held in the locations where the back surface of the main body 2 and the supporting surface are closer to each other. In other words, the main body 2 is configured to prevent the liquid L2 from becoming intermittent. Moreover, the above-mentioned configuration where the drooping member 3 is extended obliquely toward the inner side of the main body 2 and the interval to the eyelid edge E3 is narrow is also an important factor contributed to holding the liquid L2.

Suppose that the administered amount of the ophthalmic solution L1 or the amount of liquid flowing out from the eye is increased, which leads the liquid level of the liquid L2 in the palpebral fissure E5 to become higher, since the projecting member 5 of the main body 2 is projected from the eyelid margin E3 into the palpebral fissure E5, the liquid L2 can contact the back surface of the projecting member 5. Thereby, the liquid L2 is drained out of the palpebral fissure E5 by the main body 2 directly and discharged to the outside of the palpebral fissure E5. Particularly in the drainage device 1 of the first embodiment, the lateral edge 23 of the main body 2 is curved so as to match with the eyelid margin E3 in the vicinity of the lateral angle of eye E4 of a patient. Thus, even if the administered amount of the ophthalmic solution L1 or the amount of liquid flowing out from the eye is increased and consequently the liquid level of the liquid L2 in the palpebral fissure E5 becomes higher, since the liquid L2 can have a contact with the back surface of the main body 2 from the projecting member 5 along the lateral edge 23, the drawing portion for the liquid L2 expands along the lateral edge 23. Accordingly, even if the liquid level of the liquid L2 in the palpebral fissure E5 increases, the amount of liquid discharged by the main body 2 can be increased smoothly. Thereby, even if the administered amount is increased, owning to the main body 2, the liquid level of the liquid L2 is maintained without rising, which offers a clear field of operation.

As mentioned above, according to the drainage device 1 of the present invention, the liquid L2 in the palpebral fissure E5 is discharged to the outside by the use of the wettability of the projecting member 5 or the drooping member 3 of the main body 2, the liquid can be drained continuously with a simple structure made of a planar member such as paper without using the aspirator which has been used in the conventional arts. Since the liquid L2 flows between the back surface and the supporting surface of the main body 2, the resistance to the flow of the liquid L2 is smaller in comparison with the case where the gauze or sponge is used, therefore, the drainage speed is fast. In the surgical operation, oil content from the patient may be mixed into the liquid L2 to deteriorate the field of operation. According to the drainage device 1 of the present invention, since the drainage speed is fast, the oil layer on the liquid L2 originated from the oil content can be discharged efficiently. Since the projecting member 5 is projected obliquely from the eyelid margin E3 into the palpebral fissure E5, even though the ophthalmic solution L1 or the like is continuously administered to the palpebral fissure E5, the liquid can be discharged continuously to maintain the liquid level of the liquid L2 low in the palpebral fissure E5.

Even though the drainage ability of the main body 2 is decreased due to the adhesion of the liquid of high viscosity used in the surgical operation to the back surface thereof, the liquid of high viscosity can be wiped off from the back surface of the main body 2 by, for example, an assistant in the surgical operation to recover the drainage ability easily.

In the above-mentioned embodiment, the main body 2 is made of paper; therefore, the unused drooping member 3 on the other side can be fine-adjusted through cutting with a scissor or the like. In the above-mentioned embodiment, the leg member 4 is configured to be intermittent in the flowing direction of liquid; however, it is acceptable to configure it to be continuous in the flowing direction of liquid. The color of the outer surface of the drainage device 1 may be given a green color identical to the color of the non-woven fabrics used in the surgical operation, or may be given an arbitrary color in a condition that it will not apply a strange feeling to the surgery operator. The color of the back surface of the drainage device 1 may be given an arbitrary color in a condition that the illumination lights will not be reflected into the field of operation.

Hereinafter, a drainage device 1A of a second embodiment will be described with reference to Fig. 5(a) to Fig 5(c). As illustrated in Fig. 5(a), the drainage device 1A of the second embodiment is provided with a main body 2A, a drooping member 3A, and a leg member 4A which are integrally made of silicon rubber. In the second embodiment, the drooping member 3A is included in a lateral side 23A in the width direction. The vicinity of the lateral side 23A is disposed with a projecting member 5A projected into the palpebral fissure E5. As illustrated in Fig. 5(a) and Fig. 5(b), the drooping member 3A of the drainage device 1A is configured to be a column extended downward from the back surface of the main body 2A, and a lower end portion thereof is configured to have a spatula shape projected toward the downstream side of a liquid L2. The back surface of the main body 2A and the circumferential surface of the drooping member 3A are subjected to a surface treatment to apply wettability to the outer surface of the silicon rubber. As a specific example, the back surface of the main body 2A and the circumferential surface of the drooping member 3A are made rough on the outer surface, or coated with liquid of high viscosity to be used in the surgical operation to maintain intraocular pressure.

Usage of the drainage device 1A of the second embodiment is illustrated in Fig. 5(c). The drainage device 1A is disposed on the supporting surface in such a way that the drooping member 3A is made to have a contact with the eyelid margin E3 in the vicinity of the lateral angle of eye E4 or even to be sandwiched by the eyelid and the eyeball. Thereby, when the liquid L2 is pooled in the palpebral fissure E5, similar to the first embodiment, the liquid L2 contacts the drooping member 3A and the liquid level rises along the drooping member 3A. After the liquid L2 has a contact with the back surface of the main body 2A, it flows to the downstream through the gap G between the main body 2A and the outer surface of the sterile seal S.

According to the second embodiment, since the main body 2A, the drooping member 3A and the leg member 4A are made of silicon rubber, the drainage device 1A has a weight to some extent. Since the leg member 4A contacting the outer surface of the sterile seal S and the lateral side 22A in the longitudinal direction of the main body 2A are made of silicon rubber, they deform in accordance with the shape of the supporting surface; thereby, they will be well adapted to the supporting surface and have a higher friction with the supporting surface. Thus, due to the self weight of the drainage device 1A and the surface friction between the drainage device 1A and the sterile seal S, the drainage device 1A can be disposed on the outer surface of the sterile seal S stably. Furthermore, the drooping member 3A of the drainage device 1A is engaged to the eyelid margin E3. Therefore, even though a large amount of liquid L2 flows out from the palpebral fissure E5, it is not possible that the drainage device 1A will be drifted away to the downstream. It should be noted that the leg member 4A is configured to be continuous in the longitudinal direction in the second embodiment; however, it is not limited thereto, the leg member 4A may be formed from a plurality of projections. It is also acceptable to dispose a supporting column at the back surface of the main body 2A to support the main body 2A in addition to the leg member 4A. The material for forming the main body 2A is not limited to silicon rubber; it is acceptable to use the other type of rubber, elastomer, resin, metal or the like widely.

Hereinafter, a drainage device 1B of a third embodiment will be described with reference to Fig. 6(a) and Fig 6(b). The drainage device 1B of the third embodiment is provided with a main body 2B, a drooping member 3B disposed at a lower end portion of an arc-shaped lateral edge 23B of the main body 2B, and an engaging member 25 disposed at an upper end portion of the lateral edge 23B. As illustrated in Fig. 6(a), the main body 2B is provided with two mountain-fold lines 26B in the flowing direction of the liquid L2. When the main body 2B is folded along the two mountain-fold lines 26B and disposed on the eyelid or the outer surface of the sterile seal S, it forms an inclination member of a trapezoidal shape in the flowing direction of the liquid L2 with a gap G to the eyelid or the outer surface of the sterile seal S.

In the third embodiment, the drooping member 3B and the engaging member 25 may be configured to protrude to the lateral side and to be folded along folding lines into a rectangular shape with the right side open (like a capitalized letter "C") in a profile view. The lateral edge 23B of the main body 2B is formed into an arc shape in accordance with the eyelid margin E3, and the other edge 24 is formed into a linear shape. In the third embodiment, the vicinity of the lateral edge 23B including the drooping member 3B is equivalent to the projecting member 5B.

Usage of the drainage device 1B of the third embodiment is illustrated in Fig. 6(b). The drooping member 3B and the engaging member 25 are folded into the "C" shape in the profile view to engage the drainage device 1B to the eyelid margin E3. Thus, the main body 2B of the drainage device 1B according to the third embodiment is engaged to the eyelid margin E3 by the engaging member 25 and the drooping member 3B. Thereby, different from the above-mentioned embodiments, the adhesive layer 41 becomes unnecessary, making the structure of the present invention much simpler.

Hereinafter, a drainage device of a fourth embodiment will be described with reference to Fig. 7(a) and Fig 7(b). The drainage device 1C of the fourth embodiment is provided with a main body 2C, a drooping member 3C disposed at one end portion of an arc-shaped lateral edge 23C of the main body 2C, and a fixing member 27 disposed at the other end portion thereof. As illustrated in Fig. 7(b), the main body 2C is provided with one mountain-fold line 26C in the flowing direction of the liquid L2. When the main body 2C is folded along the mountain-fold line 26C and disposed on the eyelid or the outer surface of the sterile seal S, it rises in a triangular shape in the flowing direction of the liquid L2 to form a gap G. In the fourth embodiment, the drooping member 3C is incurved away from the eyelid margin E3 and the other side opposed to the curvature is cut away. In the fourth embodiment, the fixing member 27 is extended with a roughly arc-shaped curvature from the arc-shaped lateral edge 23C of the main body 2C. In the fourth embodiment, the vicinity of the lateral edge 23C including the drooping member 3C is equivalent to a projecting member 5C.

Usage of the drainage device 1C of the fourth embodiment is illustrated in Fig. 7(b). In the drainage device 1C, the disposition stability of the main body 2C is realized by sandwiching the fixing member 27 between the eyelid speculum 7 and the eyelid. According to the fourth embodiment, the lateral side of the drooping member 3C, which is close to the eyelid margin E3, is arc-shaped, the drooping member 3C is also engaged to the eyelid margin E3, therefore, the disposition stability of the main body 2C can be realized. Thereby, the adhesive layer 41 is also unnecessary in the fourth embodiment, making the structure of the present invention much simpler.

Since the drooping 3C is inclined toward the eyelid margin E3, a gap can be created with certainty between the drooping member 3C and the eyelid margin E3. Moreover, the arc shape of the drooping member 3C also forms a gap between the drooping member 3C and the eyelid margin E3. According to the above-mentioned configuration, it is easy for the capillary phenomenon to occur between the drooping member 3C and the eyelid margin E3 due to the adhesive force of the liquid L2, improving the drainage efficiency of draining the liquid L2.

Hereinafter, a drainage device of a fifth embodiment will be described with reference to Fig. 8(a) and Fig 8(b). The drainage device 1D of the fifth embodiment is provided with a main body 2D, and a drooping member 3D disposed at one end portion of an arc-shaped lateral edge 23D of the main body 2D. The drooping member 3D has the same shape and functions as the drooping member 3C of the fourth embodiment illustrated in Fig. 7(a). The main body 2D is disposed with a mountain-fold line 26D at each of the two corners at the upper edge 21D for folding each corner into a triangular shape. When the main body 2D is folded along the two mountain-fold lines 26D and disposed on the eyelid or the outer surface of the sterile seal S, it rises in a triangular shape in the flowing direction of the liquid L2 with a gap G to the eyelid or the outer surface of the sterile seal S. In the fifth embodiment, the vicinity of the lateral edge 23D including the drooping member 3D is equivalent to a projecting member 5D.

Usage of the drainage device 1D of the fifth embodiment is illustrated in Fig. 8(b). In the drainage device 1D, the drooping member 3D is engaged to the eyelid margin E3, and the lateral edge of the main body 2D to the side of the drooping member 3D is fixed with an adhesive tape T or the like commonly used in a surgical operation. Thereby, even though the main body 2D is not provided with a fixing member, since the main body 2D is made of light planar material, it can be easily fixed at an appropriate location for use by a common adhesive tape or the like. Moreover, since the two corners at the upper edge 21D of the main body 2D are folded to a triangular shape and the end portions thereof are disposed at the supporting surface, the end portions are engaged to the supporting surface. Accordingly, even without the adhesive tape or the like, the main body 2D can be supported on the supporting surface stably to a certain extent.

Hereinafter, a drainage device of a sixth embodiment will be described with reference to Fig. 9 (a) and Fig 9(b). As illustrated in Fig. 9(a), the drainage device 1E of the sixth embodiment is provided with a main body 2E, and a pair of drooping members 3E disposed at the middle of a lateral edge 23E of the main body 2E. The main body 2E is disposed with a valley-fold line 28E in the middle along the flowing direction of the liquid L2, and four mountain-fold lines 26E at four corners of the upper edge and the lower edge, respectively. An adhesive tape (not shown) is disposed along the valley-fold line 28E on the back surface opposite to the middle valley-fold line 28E. In the sixth embodiment, the vicinity of the lateral edge 23E including the drooping members 3C is equivalent to a projecting member 5E.

The drainage device 1E of the sixth embodiment has a shape like joining in parallel two drainage devices 1D of the fifth embodiment. Therefore, by fixing the drainage device 1E of the sixth embodiment as illustrated in Fig. 9(b), it can drain the liquid L2 approximately two times more than the drainage device 1D of the fifth embodiment. Thus, the drainage device 1E of the sixth embodiment is suitable for a surgical operation where a large amount of ophthalmic solution L1 is used or a large amount of liquid will flow out from the eye. The drooping member 3E is similar to a shape by joining in parallel two drooping members 3B of the third embodiment in Fig 6(a). Thereby, the drainage device 1E of the sixth embodiment can be engaged to the eyelid margin E3 by the drooping members 3E for use without an adhesive tape.

Hereinafter, a drainage device of a seventh embodiment will be described with reference to Fig. 10. The drainage device 1F of the seventh embodiment is provided with a hydrophilic membrane 29 disposed close to a lower edge 22F of the main body 2F on the back surface of the main body 2F and a drooping member 3F. The hydrophilic membrane 29 has better wettability than the other parts of the back surface of the main body 2F. The hydrophilic membrane 29 is formed by coating a coating material of high hydrophilicity on the back surface of the main body 2F and the drooping member 3F.

According to the drainage device 1F of the seventh embodiment, when the liquid L2 in the palpebral fissure E5 is firstly drained out by the drooping member 3F, since the hydrophilicity of the back surface of the drooping member 3F is improved by the hydrophilic membrane 29, the initial liquid L2 can flow out smoothly. Thereafter, when the liquid L2 drained out by the drooping member 3F flows to the downstream side of the main body 2F, the liquid L2 can also advance to the downstream side of the main body 2F smoothly according to the hydrophilic membrane 29. Even if the liquid L2 in the palpebral fissure E5 decrease, it is not possible for the liquid L2 flowing between the main body 2F and the supporting surface to become intermittent. Suppose that the liquid becomes intermittent, when the liquid L2 in the palpebral fissure E5 increases again, due to the hydrophilic membrane 29, the liquid L2 can be discharged from the palpebral fissure E5 to the outside smoothly.

In the seventh embodiment, the hydrophilic membrane 29 is formed by coating a coating material of high hydrophilicity on the back surface of the drooping member 3F and the main body 2F; however, it is not limited thereto, it is acceptable to paste a film of high hydrophilicity on the back surface of the drooping member 3F and the main body 2F. It is also to form a portion where the hydrophilic membrane 29 is disposed separate from the main body 2F and to fix the portion to the main body 2F.

It is acceptable to dispose a hygroscopic member (not shown) such as a piece of thin sponge or a piece of non-woven fabrics in place of the hydrophilic membrane 29. Similar to the hydrophilic membrane 29, the initial flow of the liquid L2 in the palpebral fissure E5 can be achieved easily by the hygroscopic member. Even though the liquid L2 between the main body 2F and the supporting surface becomes intermittent, when the liquid L2 in the palpebral fissure E5 increases again, the liquid L2 can be discharged from the palpebral fissure E5 to the outside smoothly by the hygroscopic member. When the hygroscopic member is partially disposed on the back surface of the drooping member 3F and the main body 2F, since the liquid can also flow between the supporting surface and the back surface of the main body 2F where the hygroscopic member is not disposed, the drainage speed of the liquid can be faster than the case where the liquid is discharged out by using the conventional gauze or sponge only.

Even if the hygroscopic member is soaked by the liquid of high viscosity in the surgical operation, the liquid can be discharged to the outside through the other parts of the main body 2F, the drainage ability thereof will not be extremely deteriorated as the case where the liquid is discharged out by using the conventional gauze or sponge only, instead, the adhesive force to the liquid L2 is improved. Therefore, it is acceptable to dispose the hygroscopic member in the drainage device of the present invention after the hydrophilicity of the hygroscopic member has been improved by preliminarily coating the liquid of high viscosity on the hygroscopic member.

Hereinafter, a drainage device of an eighth embodiment will be described with reference to Fig. 11. The drainage device 1G of the eighth embodiment is a variation of the drainage device 1 of the first embodiment. The lower edge 22G of the main body 2G is disposed with a plurality of slits 30G extending upward. In the present embodiment, the upper edge 21G of the main body 2G is configured to have a shape easy to be clipped by a pair of forceps or the like when the main body 2G is to be placed on or to be taken away from the supporting surface. The leg member 4G is made of flexible resin foam, and is fixed on the back surface in the vicinity of the upper edge 21G of the main body 2G by an adhesive agent. In the present embodiment, the drooping member 3G is also disposed with slits, accordingly, the flexibility of the drooping member 3G has been improved.

According to the drainage device 1G of the eighth embodiment, even if the supporting surface is uneven, since the flexibility of the main body 2G has been improved by the plurality of slits 30G disposed therein, it is easy for the main body 2G to deform in accordance with the supporting surface. Thereby, it would be difficult for the liquid L2 to become intermittent between the main body 2G and the supporting surface, enabling the liquid L2 to be drained with certainty. Moreover, since the drooping member 3G is flexible, it is also easy for the drooping member 3G to deform in accordance with the shape of the palpebral fissure E5 of the patient. If the drooping member 3G is made to have a direct contact with the conjunctiva E2 of the patient, the drooping member 3G will adhere to the conjunctiva E2, which makes it possible to hold the main body 2G stably.

Hereinafter, a drainage device of a ninth embodiment will be described with reference to Fig. 12(a) and Fig. 12(b) As illustrated in Fig. 12(a), the drainage device 1H of the ninth embodiment is provided with a main body 2H, and a pair of drooping members 3H disposed at the upper end and the lower end of a lateral edge 23H of the main body 2H, respectively. The main body 2H is further provided with one mountain-fold line 28H in the middle along the flowing direction of the liquid L2. Furthermore, the upper edge 21H and the lower edge 22H of the main body 2H are disposed respectively with a plurality of slits 30H orthogonal to the flowing direction of the liquid L2.

According to the drainage device 1H of the ninth embodiment, even if the supporting surface is uneven, since the flexibility of the main body 2H has been improved by the plurality of slits 30H disposed at the upper edge 21H and the lower edge 22H of the main body 2H, it is easy for the main body 2H to deform in accordance with the supporting surface. Thereby, it would be difficult for the liquid L2 to become intermittent between the main body 2H and the supporting surface, enabling the liquid L2 to be drained out smoothly.

As illustrated in Fig. 12(b), by displacing the position where the drooping member 3H is engaged to the palpebral fissure E5 of the patient, it is possible for the drainage device 1H of the ninth embodiment to assure an operation space for the operator. Moreover, as illustrated in Fig. 12(b), since the projecting members 5H are disposed at two locations, even if there are a large amount of liquid L2 in the palpebral fissure E5, the drainage device 1H can discharge the liquid L2 to the outside instantly. When the liquid L2 in the palpebral fissure E5 is not that much, a portion of the main body 2H close to the lower edge 22H in Fig. 12 (b) will perform the drainage mainly; however, when the amount of liquid L2 increases, a portion of the main body 2H close to the lower edge 22H will perform the drainage additionally.

Hereinafter, a drainage device of a tenth embodiment will be described with reference to Fig. 13(a) and Fig. 13(b). As illustrated in Fig. 13(a), in the drainage device 1J of the tenth embodiment, a main body 2J is split into plural parts by a plurality of slits 30J extending vertically, and the plural parts are joined together by a joining member 2Ja disposed in the middle. The main body 2J split by the slits 30J is disposed with a valley-fold line 28J in the middle along the flowing direction of the liquid L2. When viewed from the flowing direction of the liquid L2, the main body 2J has a shape of a capitalized letter "V" roughly. The middle part in the vertical direction of a lateral edge 23J of the main body 2J is disposed with a drooping member 3J. The above-mentioned members are made of flexible resin.

According to the drainage device 1J of the tenth embodiment, the main body 2J is joined by a plurality of joining members 2Ja, and is bendable at the joining members 2Ja. Therefore, as illustrated in Fig. 13(b), even if the supporting surface is uneven, it is easy for the main body 2J to deform in accordance with the supporting surface. To either side the main body 2J inclines around the valley-fold line 28J as a fulcrum, the gap G can be formed between the supporting surface and the main body 2J at the inclined side, thereby, the drainage function can be made available with certainty. It should be noted that the material used for the drainage device 1J is not limited to resin. Paper or metals subjected to water-proof processing may also be used as the material.

Hereinafter, a drainage device of an eleventh embodiment will be described with reference to Fig. 14(a) and Fig. 14(b). According to the drainage device 1K of the eleventh embodiment illustrated in Fig. 14(a), a main body 2K is provided with an inclination member 2Ka inclined relative to the supporting surface and a disposition member 2Kb to be disposed on the supporting surface integrally made of resin. A bottom view of the drainage device 1K is illustrated in Fig. 14(b). As illustrated in Fig. 14(b), a projecting member 5K projecting inward from the edge portion of the palpebral fissure E5 is configured to project into the eyelid with a greater inclination angle than the disposition member 2Kb. A back surface side of the disposition member 2Kb is formed with an adhesive portion to be adhered to the supporting surface. A drooping member 3K of the present embodiment is disposed at an end portion of the back surface of the disposition member 2Kb of the main body 2K, formed integrally with the main body 2K. The drooping member 3K is configured to form a surface facing the lateral angel of eye E4 into a roughly half circular shape so as to be easily engaged to the lateral angle of eye E4. In order to drain the liquid L2 from the palpebral fissure E5 efficiently, a cut-off 3Ka of a roughly triangular shape is disposed. It should be noted that it is not necessary to form the disposition member 2Kb and the inclination member 2Ka of the main body 2K integrally. For example, both are formed separately, a slit is disposed in the vicinity of an end edge of the disposition member 2Kb; the two members are fixed together by inserting the slit into an end edge of the inclination member 2Ka.

According to the drainage device 1K of the eleventh embodiment, even though the supporting surface is uneven, since the disposition member 2Kb of the main body 2K is adhered to the supporting surface, the main body 2K can be fixed firmly on the supporting surface with certainty. Further, since the shape of the drooping member 3K is formed in accordance with the shape of the lateral angle of eye E4, the main body 2K can be fixed closely on the supporting surface with certainty. When the drainage device 1K is disposed on the supporting surface in the vicinity of the lateral angle of eye E4, the liquid L2 in the palpebral fissure E5 is drained out through the cut-off 3Ka of the drooping member 3K and is discharged to the outside through the gap between the disposition member 2Kb and the inclination member 2Ka of the main body 2K. According to the drainage device 1K of the present embodiment, the disposition member 2Kb and the inclination member 2Ka are integrally formed, the gap in the joint portion of the disposition member 2Kb and the inclination member 2Ka is narrow and the gap becomes wider when it goes away from the joint portion. Therefore, when the liquid L2 in the palpebral fissure E5 is of a small amount, it flows through the joint portion of the disposition member 2Kb and the inclination member 2Ka; when the liquid L2 in the palpebral fissure E5 is of a great amount, it flows by spanning to a location away from the joint portion. Thereby, despite of the amount of the liquid L2, the liquid L2 can be discharged to the outside from the palpebral fissure E5 smoothly.

Hereinafter, variation examples of the drooping member in the drainage device of the present invention will be described. As illustrated in Fig. 15(a), the drooping member 31 has a triangular shape formed by folding the main body 2 along a mountain-fold line 31A disposed at a lower left corner thereof. Even with such a simple configuration, since a tip end of the drooping member 31 extends into the palpebral fissure E5, it is possible to drain the liquid L2 pooled in the palpebral fissure E5 according to the wettability of the back surface of the drooping member 31.

The drooping member 32 illustrated in Fig. 15(b) includes two parts 32A and 32B extending in parallel from the main body 2 with a predefined interval therebetween. With the mentioned configuration, the adhesive force and the capillary phenomenon between the parts of the drooping member 32 are increased; therefore, the liquid L2 pooled in the palpebral fissure E5 can be discharged to the outside with certainty even better. The configuration and function of each part, for example, the part 32A, are similar to the drooping member 3C in the fourth embodiment of the present invention.

The drooping member 33 illustrated in Fig. 15(c) is made of three parts mold integrally with the main body 2 from resin. By providing a plurality of parts in parallel with a predefined interval therebetween, the adhesive force and the capillary phenomenon between the parts are increased; therefore, the liquid L2 pooled in the palpebral fissure E5 can be discharged to the outside with certainty even better. The lower portions of the drooping member 33 composed of the plurality of parts may be jointed.

The drooping member 34 illustrated in Fig. 15(d) is made of a cutting-plane line 34A, four mountain-fold lines 34B and one valley-fold line 34C disposed in the main body 2. According to the folding lines, the drooping member 34 is made of two triangular mountain-like parts arranged in parallel. By configuring the drooping member 34 with the mentioned structure, the adhesive force and the capillary phenomenon with respect to the liquid L2 pooled in the palpebral fissure E5 are increased due to the gap generated between the drooping member 34 and the eyelid margin E3, the drainage of the liquid L2 can be improved effectively.

The drooping member 35 illustrated in Fig. 15(e) is made of silicon rubber together with the main body 2. The drooping member 35 is composed of a prism part 35A drooping from the main body 2 and an engaging plate 35B disposed at a lower end portion of the prism part 35A. The outer surface of the drooping member 35 is subjected to a surface treatment for assuring wettability. The main body 2 can be disposed on the supporting surface stably by inserting the drooping member 35 into the palpebral fissure E5, by sandwiching it between the eyelid and the eyeball, or by engaging the prism part 35A and the engaging plate 35B to the eyelid margin E3. Due to the wettablity of the outer surface of the prism part 35A and the engaging plate 35B, the liquid L2 in the palpebral fissure E5 can be discharged to the outside.

The drainage device in each embodiment and each variation example mentioned above is configured to have a size to be able to be placed inside the supporter 71 of a roughly "C" shape disposed in the eyelid speculum 7. Therefore, the drainage device of the present invention can be easily disposed for use after the eyelid speculum 7 has been set on the patient.

In each embodiment and each variation example mentioned above, the main body 2 or the drooping member 3 made of paper may also be made of hard or soft resin or metals. The drainage device may be used in a single unit or a plurality of drainage devices are used in an overlapped state. The drainage device of the present invention is described by using the eyelid speculum 7 disposed with the supporter 71 of a roughly "C" shape which is widely used in the conventional arts; however, it is not limited thereto, the drainage device of the present may also be applicable when the eyelid is held by an eyelid speculum of the other shape. For example, in the case where a flexible tubular eyelid speculum which is invented in accordance with the shape of eye and is provided with a portion inserted into the palpebral fissure E5 and a portion for covering the skin in the ambient of the eye is used, by disposing the drainage device of the present invention on the portion covering the skin in the ambient of the eye, the liquid L2 in the palpebral fissure E5 can be discharged to the outside.

## Claims

1. A drainage device used in an ophthalmic surgery to drain a liquid to the outside from a palpebral fissure of a patient lain with face up, comprising:
a planar main body configured to be disposed with a predefined gap on a supporting surface on an eyelid in the vicinity of a lateral angle of eye;
the main body is made of a water-proof material and has a wettable back surface;
the main body is provided with a projecting member projected from an edge of the palpebral fissure into an inner side thereof; and
when the main body is disposed on the supporting surface, the liquid pooled in the palpebral fissure is adhered by a back surface of the projecting member and is drained into the gap between the main body and the supporting surface, and the drained liquid flows through the gap to be discharged to the outside of the main body.

2. The drainage device for ophthalmic surgery according to claim 1, wherein the projecting member is provided with a drooping member drooped toward the conjunctiva in the vicinity of the lateral angle of eye.

3. The drainage device for ophthalmic surgery according to claim 1 or 2, wherein a part of or the entire part of the main body is inclined to the supporting surface when viewed from the flowing direction of the liquid.

4. The drainage device for ophthalmic surgery according to claim 3, wherein the main body protrudes in a mountain-like shape when viewed from the flowing direction of the liquid.

5. The drainage device for ophthalmic surgery according to claim 3, wherein the main body has a capitalized letter "V" shape when viewed from the flowing direction of the liquid.

6. The drainage device for ophthalmic surgery according to any of claims 1 to 5, wherein the main body is provided with a plurality of slits orthogonal to the flowing direction of the liquid.

7. The drainage device for ophthalmic surgery according to any of claims 1 to 6, wherein the main body is provided with a leg member of a predefined height on the back surface thereof.

8. The drainage device for ophthalmic surgery according to any of claims 1 to 7, wherein an edge of the projecting member provided in the main body is curved in accordance with the eyelid margin.

9. The drainage device for ophthalmic surgery according to any of claims 1 to 8, wherein the back surface of the main body is provided with a hydrophilic membrane toward the flowing direction of the liquid with better wettability than the other surface thereof.

10. The drainage device for ophthalmic surgery according to any of claims 1 to 9, wherein the back surface of the main body is provided with a hygroscopic member toward the flowing direction of the liquid.

11. The drainage device for ophthalmic surgery according to any of claims 1 to 10, wherein the main body is provided with a fixing member protruded to the vicinity of the eyelid margin to be fixed on the eyelid.

12. The drainage device for ophthalmic surgery according to any of claims 1 to 10, wherein the main body is provided with an engaging member protruded to the palpebral fissure to be engaged to the eyelid margin.

13. The drainage device for ophthalmic surgery according to any of claims 1 to 12, wherein the main body is provided with an adhesive member to be adhered to the supporting surface.

14. The drainage device for ophthalmic surgery according to any of claims 1 to 13, wherein the main body is composed of a holding member to be fixed on the supporting surface and an inclination member inclined obliquely upward from the holding member.

15. The drainage device for ophthalmic surgery according to any of claims 1 to 14, wherein a portion of the drooping member opposite to the eyelid margin is inclined toward the eyelid margin when the main body is disposed on the supporting surface.

16. The drainage device for ophthalmic surgery according to any of claims 1 to 15, wherein a plurality of drooping members are disposed in parallel with an interval therebetween appropriate for giving birth to a capillary phenomenon between the plurality of drooping members.

17. The drainage device for ophthalmic surgery according to any of claims 1 to 16, wherein the drooping member is formed with an upstream side and a downstream side symmetrical at the central position of the main body in a longitudinal direction identical to the flowing direction of the liquid.

18. The drainage device for ophthalmic surgery according to any of claims 1 to 17, wherein the main body is formed to have a size to be disposed inside a capitalized letter "C" shaped supporting member of an eyelid speculum.
